Europäisches Patentamt

European Patent Office

Office européen des brevets

(11) Publication number: **0 077 189**
**B1**

(12) **EUROPEAN PATENT SPECIFICATION**

(45) Date of publication of patent specification: **12.08.87**

(21) Application number: **82305362.4**

(22) Date of filing: **08.10.82**

(51) Int. Cl.⁴: **A 61 M 1/02,** A 61 M 5/14, A 61 J 1/00

(54) **An infusion unit.**

(30) Priority: **09.10.81 ZA 817007**

(43) Date of publication of application:
**20.04.83 Bulletin 83/16**

(45) Publication of the grant of the patent:
**12.08.87 Bulletin 87/33**

(84) Designated Contracting States:
**AT BE CH DE FR GB IT LI LU NL SE**

(56) References cited:
**AT-B- 193 071
AT-B- 198 424
CH-A- 550 007
DE-A- 2 006 054
US-A- 2 766 907
US-A- 2 940 444
US-A- 3 023 750
US-A- 3 054 401
US-A- 4 041 944
US-A- 4 270 533**

(73) Proprietor: **BIOFUSION (PROPRIETARY) LIMITED**
**49 Lilian Avenue Fordsburg**
**Johannesburg Transvaal (ZA)**

(72) Inventor: **Edge, Kenneth Roger**
**15 Ravenswood Avenue**
**Birdhaven Transvaal (ZA)**

(74) Representative: **Boon, Graham Anthony et al**
**Elkington and Fife High Holborn House 52/54**
**High Holborn**
**London, WC1V 6SH (GB)**

EP 0 077 189 B1

Courier Press, Leamington Spa, England.

## Description

The present invention relates to a infusion unit for blood, blood products and intravenous solutions.

Certain types of units are already known such as those disclosed in AT—B—198424 and US—A—4270533 which comprise a container including a deformable first bag located within a deformable second bag, the first bag being formed from a pair of flexible sheets of polymer material which are sealed together about their peripheries and over a first tube communication means for charging and discharging an infusion liquid into and from said first bag and said second bag being formed from a pair of flexible sheets of polymer material which are sealed together at their peripheries outwardly with respect to the seal of the first bag and over the first tube communication means as well as over second tube communication means for charging the second bag with a pressurizing fluid whereby the first bag is subjected to extreme pressure for collapsing the first bag and thereby discharging the infusion liquid therefrom, the seals of the first and second bags on the first tube communication means providing an anchor zone for the bags with respect to one another.

According to the present invention there is provided an infusion unit as described above characterised in that the first and second bags are separate from one another and engage sealingly over the first tube communication means in axially spaced zones thereof.

The first communication means may include a single inlet and outlet port in the first bag for the passage of the infusion liquid, the port being communicable with a suitable inlet and outlet port alternately. Instead, the first communication means may include a plurality of ports in the first bag, at least one constituting an inlet port and at least one constituting an outlet port for the passage of the infusion liquid, the or each inlet and outlet ports being communicable with a suitable inlet and outlet system respectively. Where separate inlet and outlet systems are provided, they may each include different coloured tubing to more easily differentiate therebetween.

The or each inlet and/or outlet ports of the first bag may have a filter for filtering the infusion liquid entering and/or leaving the first bag. Further, where separate inlet and outlet ports are provided, the or each inlet port may be provided with a non-return valve.

The second communication means may include a port in the second bag for the passage of a fluid into and out of the second bag. The port may be communicable with a pump for pumping the fluid into the second bag to pressurise the second bag and thereby cause the first bag to decrease in volume. The infusion liquid in the first bag is thereby expelled through the outlet port.

Tubing of a different colour to that used for the inlet/outlet systems of the first bag may be used to connect the port of the second bag to the pump, to more easily differentiate therebetween.

The infusion unit may be used as a collection bag for collecting the infusion liquid. The infusion liquid may be stored therein, and may subsequently be pumped directly therefrom for infusion into a recipient.

Instead the infusion unit may be used as an inline transfusion device. For this application the first bag will have both an inlet and an outlet port. In use the inlet port will communicate with a collection/storage bag containing the infusion liquid, and the outlet port will be in communication with a recipient of one liquid via the outlet system mentioned above.

In order to facilitate filling the first bag with the infusion liquid, the outer surfaces of the unit's walls may be provided with an appropriately located gripping means for enabling the walls to be pulled away from each other, and thus to create a partial vacuum in the first bag.

The invention is now described by way of example, with reference to the accompanying drawings in which:

Figure 1 is a side view of an infusion unit which is not an embodiment of the invention but which has some features of the infusion unit according to the invention;

Figure 2 is a schematic longitudinal section through an infusion unit according to the invention.

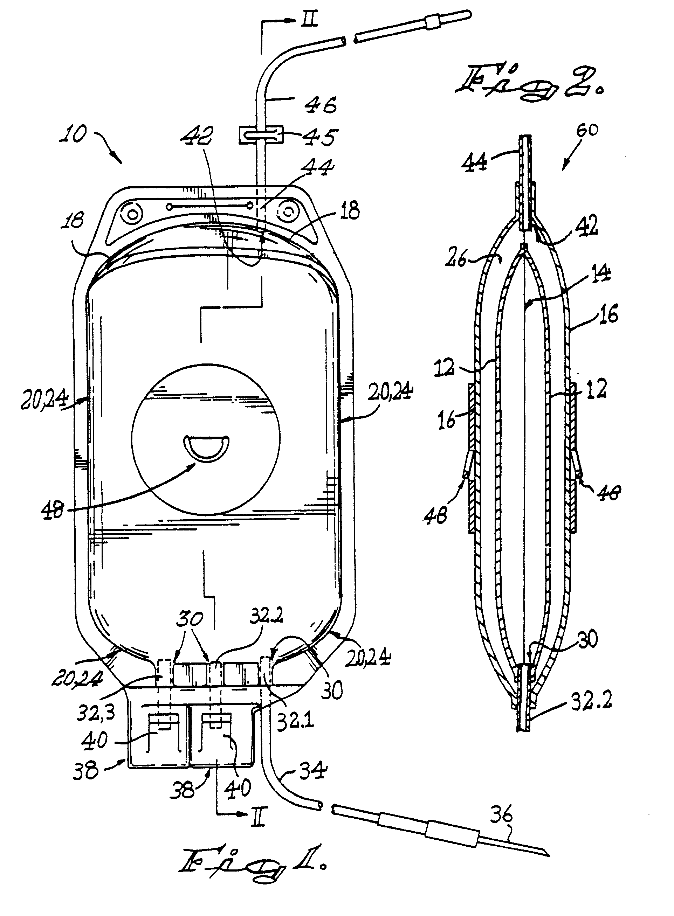

In Figure 1 reference numeral 10 generally indicates an infusion unit for blood with some of the main features of the infusion unit according to the invention. In Figure 2 reference numeral 60 shows an embodiment of the infusion unit according to the invention.

The infusion units 10 and 60 include two first sheets 12 of a flexible plastics material, that are peripherally secured to one another to define a first bag for the blood (not shown in the drawings).

The unit 10 further includes two larger sheets 16 of the flexible plastics material, that are secured to one another along a portion 18 of their peripheries and to the first sheets 12 along the rest 20 of their peripheries. The larger sheets 16 are secured to the first sheets 12 along a peripheral portion 24 of the latter. It should be understood that this particular feature of the infusion unit 10 is not a feature of the infusion unit 60 according to the invention.

In the infusion unit 60 the larger sheets 16 and the first sheets 12 located therebetween, define a second compartment 26. Thus the first sheets 12 form a common wall between a first compartment 14 and the second compartment 26. The larger sheets 16 of the infusion unit 60 are secured to one another along their entire peripheries and are not secured at all to the first sheets 12.

The infusion units 10 and 60 further include a first communication means for charging and discharging the infusion liquid into and from the first compartment 14 respectively, and a second communication means for charging the second compartment 26 with air.

The infusion unit 60 is similar to the infusion unit 10 in all respects except that the larger sheets 16 of the infusion unit 60 are secured to one another along their entire peripheries and are not secured at all to the sheets 12.

The first communication means includes three adjacent ports 30 in the first compartment 14, each port having an inlet tube 32.1 and two outlet tubes 32.2 and 32.3, all of a plastics material, extending a short distance therefrom. The inlet and outlet tube walls are sufficiently rigid to maintain an open tubular shape in normal use.

In Figure 1 the inlet tube 32.1 has one end of a flexible plastics receiving tube 34 located therein. The free end of the receiving tube 34 is fitted with a hollow needle 36.

Each of the outlet tubes 32.2 and 32.3 is located in a sealed envelope 38 having an integral pull tab 40 for opening the envelope 38, to expose the free end of the outlet tube 32.2 or 32.3.

The second communication means includes a port 42 in the second bag, for the passage of air in and out of the bag 16. The port 42 is fitted with a short inlet/outlet tube 44 in which one end of a flexible plastics air tube 46 is sealed. In use, the port 42 communicates via the air tube 46 with a pump (not shown in the drawings) for pumping air into the second bag 16.

In order to facilitate filling the first bag with blood, the outer surface of each of the larger sheets 16 is provided with a gripping formation 48 for enabling the sheets 16, to be pulled away from each other using a clamp 45 to close off the tube 46. Thus a partial vacuum is created in the second bag 16 which in turn creates a partial vacuum in the first bag.

In use, as shown in Figure 1 the first bag of the infusion unit 10 is filled with blood (not shown in the drawings) from a donor via the hollow needle 36, the receiving tube 34, and the inlet tube 32.1. When the first bag contains a predetermined volume of blood, the first bag is suitably sealed. The blood is stored in the unit 10 until required.

When the blood is required, one of the envelopes 38 is opened via the pull tab 40, to expose the free end of a first of the outlet tubes 32.2 or 32.3, which is then placed in communication with a blood recipient via any suitable outlet system. The second of the outlet tubes 32.2 or 32.3 is provided merely as a spare.

Air is pumped into the second bag via the port 42, thus pressurizing the second bag and causing the first bag 12 to decrease in volume. The blood in the first bag is expelled through the outlet tube 32.2.

Alternatively the first communication means can comprise of a single port 32.2 in the first bag for both charging and discharging of the infusion liquid.

Advantages of the infusion unit of the invention, at least as exemplified, include its versatility of application as a receiving bag and/or storage bag for an infusion liquid, and as an infusion liquid supply or inline device, for use in conjunction with a pump described. Further, the infusion unit of the invention is safe, simple, effective and relatively inexpensive, having a negligible incidence of sepsis and haemolysis of blood cells.

## Claims

1. An infusion unit (60) comprising a container including a deformable first bag located within a deformable second bag, the first bag being formed from a pair of flexible sheets of polymer material (12) which are sealed together about their peripheries and over a first tube communication means (32.2) for charging and discharging an infusion liquid into and from said first bag, and said second bag being formed from a pair of flexible sheets of polymer material (16) which are sealed together at their peripheries outwardly with respect to the seal of the first bag and over the first tube communication means as well as over second tube communication means (44) for charging the second bag with a pressurizing fluid whereby the first bag is subjected to external pressure for collapsing the first bag and thereby discharging the infusion liquid therefrom, the seals of the first and second bags on the first tube communication means (32.2) providing an anchor zone for the bags with respect to one another characterised in that the first and second bags are separate from one another and engage sealingly over the first tube communication means (32.2) in axially spaced zones thereof.

2. The infusion unit of claim 1 in which the bags are separate in the zone of the second communication means (44).

3. An infusion unit as claimed in claim 1 or 2 wherein the first communication means includes a plurality of ports (32.1, 32.2, 32.3) in the first bag, at least one constituting an inlet port and at least one constituting an outlet port (30) for the passage of infusion liquid.

4. An infusion unit as claimed in any one of claims 1 to 3, wherein the second communicating means (44) includes a port (42) in the second bag for the passage of a fluid into and out of the second bag.

5. The infusion unit of any of claims 1 to 4, in which the second bag is equipped with an external pull tab (48) on each of two opposite sides thereof.

## Patentansprüche

1. Infusionsvorrichtung (60) mit einem Behälter mit einem deformierbaren ersten Beutel, der innerhalb eines deformierbaren zweiten Beutels angeordnet ist, wobei der este Beutel aus einem Paar von flexiblen Blättern aus einem polymeren Material (12) gebildet ist, die an ihren äußeren Umfängen und über einer ersten Röhrenverbindungsvorrichtung (32.2) verschlossen sind zum Laden und Entladen einer Infusionsflüssigkeit in den ersten Beutel hinein und aus ihm heraus, und der zweite Beutel aus einem Paar von flexiblen Blättern (16) gebildet ist, die an ihren äußeren Umfängen außerhalb in Bezug auf den Verschluß des ersten Beutels und über der ersten Röhrenverbindungseinrichtung als auch über einer zweiten Röhrenverbindungseinrichtung (44) miteinander verschlossen sind zum Laden des zweiten Beutels mit einer druckbeaufschlagenden Flüssigkeit, wobei der erste Beutel einem externen Druck ausgesetzt wird zum Zusammendrücken des ersten Beutels und dadurch Entladen der Infusionsflüssigkeit daraus, die Verschlüsse des ersten und zweiten Beutels auf der ersten Röhrenverbindungsvorrichtung als Verankerungszone für die Beutel im Verhältnis zueinander vorgesehen sind, dadurch gekennzeichnet, daß der erste und zweite Beutel voneinander getrennt sind und verschließend über die erste Röhrenverbindungsvorrichtung (32.2) in axial in einem Abstand voneinander angeordneten Zonen darauf angreifen.

2. Infusionsvorrichtung nach Anspruch 1, in der die Beutel in der Zone der zweiten Verbindungsvorrichtung (44) getrennt sind.

3. Infusionsvorrichtung nach Anspruch 1 oder 2, in der die erste Verbindungsvorrichtung eine Mehrzahl von Öffnungen (32.1, 32.2, 32.3) in dem ersten Beutel aufweist, von denen mindestens eine eine Einlaßöffnung darsellt und mindestens eine eine Auslaßöffnung (30) darstellt für den Durchgang der Infusionsflüssigkeit.

4. Infusionsvorrichtung nach einem der Ansprüche 1 bis 3, in der die zweite Verbindungsvorrichtung (44) eine Öffnung (42) in dem zweiten Beutel für den Durchgang einer Flüssigkeit in den Beutel hinein und aus ihm heraus aufweist.

5. Infusionsvorrichtung nach einem der Ansprüche 1 bis 4, in dem der zweite Beutel mit einem äußeren Zugansatz (48) auf jeder von zwei sich gegenüberliegenden Seiten davon versehen ist.

**Revendications**

1. Dispositif de perfusion (60) comprenant un réservoir comportant un premier sac déformable disposé à l'intérieur d'un second sac déformable, le premier sac étant formé par une paire de feuilles souples de matière polymère (12) qui sont scellées ensemble à leur périphérie et par-dessus un premier moyen de communication à tube (32.2) destiné à introduire un liquide de perfusion dans le premier sac et à l'en évacuer, et le second sac étant formé par une paire de feuilles souples de matière polymère (16) qui sont scellées ensemble au niveau de leur périphérie, vers l'extérieur par rapport au joint du premier sac, et par dessus le premier moyen de communication à tube, ainsi que par-dessus un second moyen de communication à tube (44) permettant d'introduire dans le second sac un fluide de mise sous pression à l'aide duquel le premier sac est soumis à une pression extérieure permettant d'écraser ce premier sac et d'évacuer ainsi de celui-ci le liquide de perfusion, les joints des premier et second sacs situés sur le premier moyen de communication à tube (32.2) fournissant une zone d'ancrage des sacs l'un par rapport à l'autre, caractérisé en ce que les premier et second sacs sont séparés l'un de l'autre et viennent en appui de manière étanche sur le premier moyen de communication à tube (32.2) dans des zones de celui-ci espacées dans le sens axial.

2. Dispositif de perfusion selon la revendication 1, dans lequel les sacs sont séparés dans la zone du second moyen de communication (44).

3. Dispositif de perfusion telle que revendiqué dans la revendication 1 ou 2, dans lequel le premier moyen de communication comprend plusieurs orifices (32.1, 32.2, 32.3) ménagés dans le premier sac, au moins l'un de ceux-ci constituant un orifice d'entrée et au moins un constituant un orifice de sortie (30) pour le passage du liquide de perfusion.

4. Dispositif de perfusion telle que revendiqué dans l'une des revendications 1 à 3, dans lequel le second moyen de communication (44) comprend un orifice (42) ménagé dans le second sac pour le passage d'un fluide dans ce second sac et hors de celui-ci.

5. Dispositif de perfusion suivant l'une des revendications 1 à 4, dans lequel le second sac est équipé d'une languette extérieure de traction (48) sur chacune de ses deux faces opposées.

Fig 2.

Fig 1.